# EUROPÄISCHE PATENTANMELDUNG

(11) **EP 1 203 780 A1**
(43) Veröffentlichungstag der Anmeldung: **08.05.2002**
(21) Anmeldenummer: 01125317.6
(22) Anmeldetag: 26.10.2001
(51) Int. Cl.: C08G 18/71, C07C 263/02, C07C 265/02

(54) **Isocyanate mit Aminogruppen**

(30) Priorität: 06.11.2000 DE 10054934
(71) Anmelder: BAYER AG, 51368 Leverkusen (DE)
(72) Erfinder: Podszun, Wolfgang, Dr., 51061 Köln (DE); Krüger, Joachim, 40789 Monheim (DE); Karlou-Eyrisch, Kamelia, Dr., 40235 Düsseldorf (DE)

(57) **Zusammenfassung**

Die Erfindung betrifft neue Isocyanate mit tert. Aminogruppen, ein Verfahren zu Ihrer Herstellung, sowie ihre Verwendung zur Synthese und Modifizierung von Polymeren.

Gegenstand der Erfindung sind Isocyanate der Formel (I) 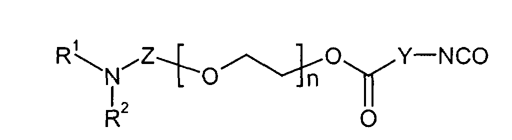 worin
- R¹ und R²: unabhängig von einander Alkyl mit 1 bis 6 C-Atomen,
wobei R¹ und R² zu einem Ring verbunden sein können,
- Z: Alkylen mit 2 bis 6 C-Atomen
- n: eine ganze Zahl von 0 bis 20
- Y: Alkylen mit 2 bis 6 C-Atomen bedeuten.

Die Alkylreste R¹ und R² können linear oder verzweigt oder zu einem aliphatischen Ring, vorzugsweise zu einem fünfgliedrigen oder sechsgliedrigen Ring verbunden sein. Bevorzugt sind Alkylreste mit 1 bis 4 C-Atomen, besonders bevorzugt ist Methyl.

Der Alkylenrest Z enthält vorzugsweise 2 bis 4 C-Atome, besonders bevorzugt 2 C-Atome. n bedeutet vorzugsweise 0 bis 10, besonders bevorzugt 1 bis 6.

## Beschreibung

Die Erfindung betrifft neue Isocyanate mit tert. Aminogruppen, ein Verfahren zu Ihrer Herstellung, sowie ihre Verwendung zur Synthese und Modifizierung von Polymeren.

Gegenstand der Erfindung sind Isocyanate der Formel (I) 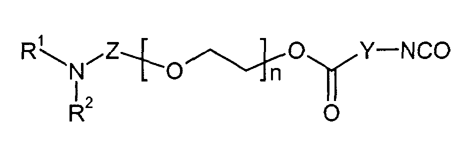 worin
- R¹ und R²: unabhängig von einander Alkyl mit 1 bis 6 C-Atomen,
wobei R¹ und R² zu einem Ring verbunden sein können,
- Z: Alkylen mit 2 bis 6 C-Atomen
- n: eine ganze Zahl von 0 bis 20
- Y: Alkylen mit 2 bis 6 C-Atomen bedeuten.

Die Alkylreste R¹ und R² können linear oder verzweigt oder zu einem aliphatischen Ring, vorzugsweise zu einem fünfgliedrigen oder sechsgliedrigen Ring verbunden sein. Bevorzugt sind Alkylreste mit 1 bis 4 C-Atomen, besonders bevorzugt ist Methyl.

Der Alkylenrest Z enthält vorzugsweise 2 bis 4 C-Atome, besonders bevorzugt 2 C-Atome. n bedeutet vorzugsweise 0 bis 10, besonders bevorzugt 1 bis 6.

Bevorzugt sind Isocyanate der Formel (II) 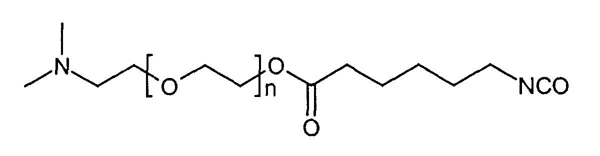 Mit n = 0 bis 20, vorzugsweise 0 bis 10, besonders bevorzugt 1 bis 6

Beispiele für erfindungsgemäße Isocyanate sind die nachfolgenden Verbindungen:

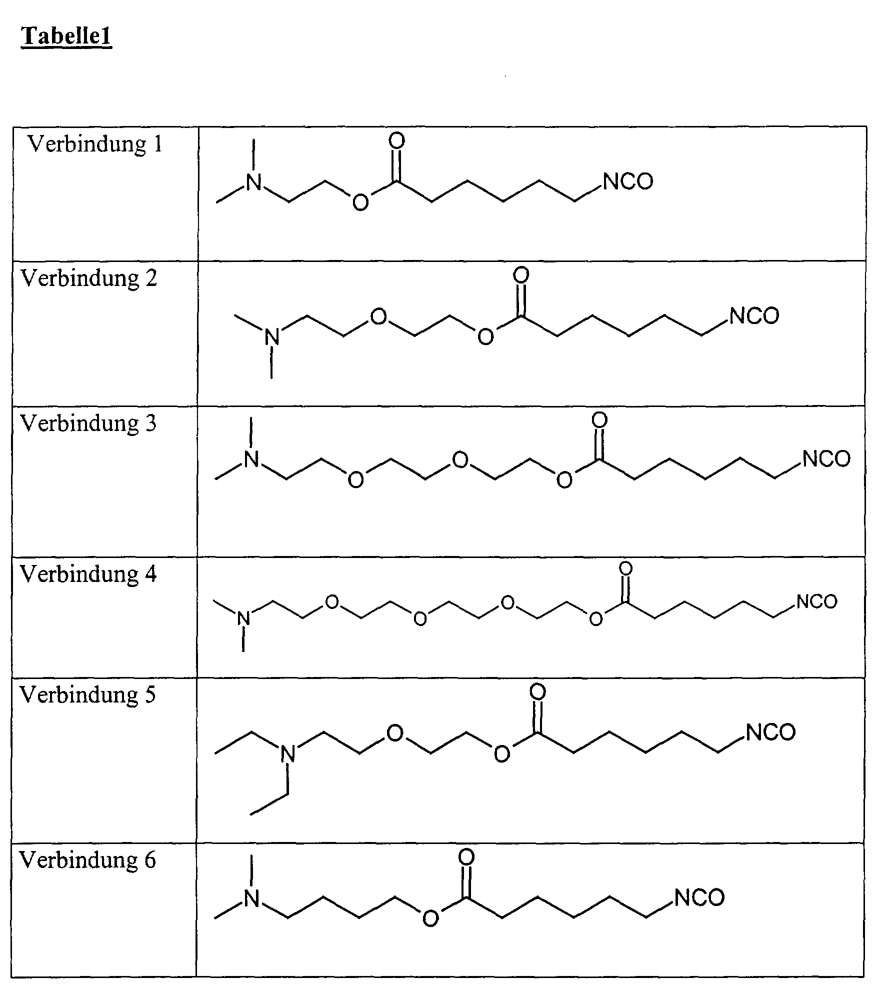

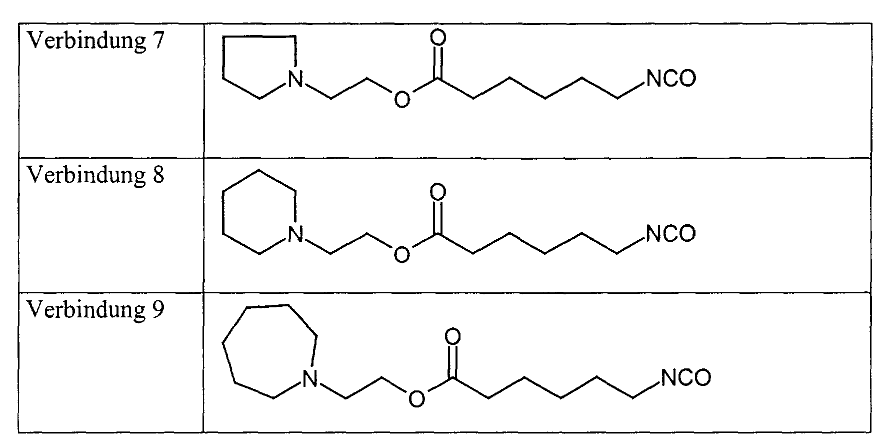

Ein weiterer Gegenstand der vorliegenden Erfindung ist ein Verfahren zur Herstellung von Isocyanaten gemäß Formel (I), welches dadurch gekennzeichnet ist, dass man Silylverbindungen der Formel (III) 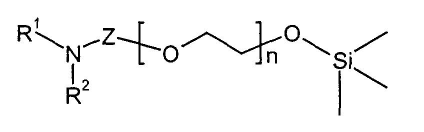 mit Isocyanatocarbonsäurechloriden der Formel (IV) umsetzt, 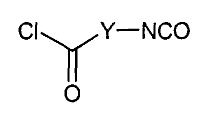 wobei R¹, R², Z, n, und Y die oben angegebenen Bedeutungen haben.

Silylverbindungen der Formel (III) können durch Silylierung der entsprechenden Hydroxylverbindungen erhalten werden. Geeignete Hydroxylverbindungen sind zum Beispiel 2-Dimethylaminoethanol, 2-Diethylaminoethanol, 2-Dioctylaminoethanol, 3-Dimethylaminopropanol, 3-Diethylaminopropanol, 3-Dibutylaminopropanol, 4-Dimethylaminobutanol, 4-Diethylaminobutanol, 5-Dimethylaminopentanol, 6-Dimethylaminohexanol, N-(2-Hydroxyethyl)pyrrolidin, N-(2-Hydroxyethyl)piperidin, N-(3-Hydroxypropyl)piperidin, N-(2-Hydroxyethyl)hexamethylenimin, sowie deren Ethoxylierungsprodukte. Trimethylchlorsilan ist ein erfindungsgemäß gut geeignetes Silylierungsmittel. Gut geeignet sind auch Mischungen von Trimethylchlorsilan und Hexamethyldisilazan. Die zugrunde liegenden Hydroxyverbindungen mit n = 1 bis 20 können auf an sich bekannte Weise durch Ethoxylierung erhalten werden. Es hat sich gezeigt, dass zur Isolierung und Reinigung der Ethoxylierungsprodukte die Anwendung von stark sauren Ionenaustauschern sehr gut geeignet ist.

Isocyanatocarbonsäurechloriden der Formel (IV) sind bekannt und können beispielsweise durch Umsetzung von Aminocarbonsäuren mit Phosgen hergestellt werden. Weitere Einzelheiten der Synthese werden in W. Mormann, S. Hoffmann, W. Hoffmann, Chemische Berichte **120**, 285-290 (1987) und in der DE-A 2 120 090 beschrieben.

Die Umsetzung der Silylverbindung der Formel (III) mit dem Isocyanatocarbonsäurechlorid der Formel (IV) kann ohne Lösungsmittel in Substanz durchgeführt werden. Die Reaktion ist exotherm und wird vorzugsweise bei niedriger Temperatur von beispielsweise 0° bis 30°C begonnen. Zur Kontrolle des exothermen Reaktionsverlaufes ist es günstig, wenn eine der Reaktionskomponenten, vorzugsweise das Isocyanatocarbonsäurechlorid, über einen Zeitraum von 10 bis 100 min. zudosiert wird. Nach Ende der Zugabe wird die Reaktionstemperatur gesteigert, beispielsweise auf 80 bis 150°C. Zur Entfernung des entstehenden Trimethylchlorsilan kann ein leichtes Vakuum von 50 bis 500 mbar angewendet werden. Der Umsatz lässt sich durch eine quantitative Bestimmung des freigesetzten Trimethylsilylchlorids kontrollieren.

Die erfindungsgemäßen Isocyanate können mit Hydroxy- und Amino-Verbindungen (NH₂ oder NH) zu Urethanen bzw. Harnstoffen umgesetzt werden. Die Anwendung eines Katalysators ist dabei in der Regel nicht notwendig. Sofern gewünscht können jedoch bekannte Katalysatoren wie z. B. Dibutylzinnlaurat, Triphenylstibin oder Triphenyphosphin verwendet werden. Die Katalysatoren werden dann in Mengen von 50 bis 5000 ppm bezogen auf die Edukte eingesetzt.

Besonders geeignet sind die erfindungsgemäßen Isocyanate zur Durchführung von polymeranalogen Umsetzungen. So lassen sich in vorhandene Polymere mit OH- oder NH₂ in einfacher Weise tert. Aminogruppen einführen. Es können auch partikelförmige, gegebenenfalls vernetzte Polymerisate, beispielsweise Perlpolymerisate mit den erfindungsgemäßen Isocyanate umgesetzt bzw. modifiziert werden. Auch die Modifizierung von magnetischen, insbesondere superparamagnetischen Perlpolymerisaten ist möglich. Für die Reaktion mit vernetzten Perlpolymerisates wird zur Erzielung hoher Umsätze ein Quellmittel, wie Chloroform, Methylenchlorid oder Methylethylketon angewendet.

Perlpolymerisate, die mit den erfindungsgemäßen Isocyanaten modifiziert wurden sind für zahlreiche Anwendungen einsetzbar. Beispielhaft seien die Verwendung als Ionenaustauscher, als Festphase in der Chromatographie oder zur Isolierung von Nukleinsäuren in der Diagnostik genannt.

### Beispiel 1

### Umsetzung von Dimethylaminoethanol mit Ethylenoxid

887 g Dimethylaminoethanol (DMAE) wurden unter Stickstoff in einen 51 VA-Druckreaktor eingewogen und auf 120°C erwärmt. Innerhalb von 6 h wurden portionsweise insgesamt 1535 g Ethylenoxyd (EO)aufgedrückt. Nach Abkühlen und entspannen wurden 2031 g eines gefärbten viskosen Gemisches von ethylenoxidverlängertem Dimethylaminoethanol erhalten.

Das Gemisch wurde nun über eine 1,5 m lange Silbermantelkolonne mit Kolonnenkopf, im Hochvakuum bei einer Badtemperatur von 70 bis 240°C destillativ aufgereinigt. Es wurden 3 Fraktionen mit konstanten Siedepunkten erhalten.

| | |
|---|---|
| Fraktion 1 | 387 g, Siedepunkt bei 0,15 mbar 45°C, DMAE x 1EO ( Identifizierung durch Elementaranalyse und NMR-Spektrum) |
| Fraktion 2 | 319 g, Siedepunkt bei 0,15 mbar 70°C, DMAE x 2EO ( Identifizierung durch Elementaranalyse und NMR-Spektrum) |
| Fraktion 3 | 128 g, Siedepunkt bei 0,15 mbar 110°C, DMAE x 3EO ( Identifizierung durch Elementaranalyse und NMR-Spektrum) |

Zur weiteren Reinigung der Fraktionen 1, 2 und 3 wurden jeweils 15 g Produkt in eine Säule gegeben, die mit 200 ml Wasser und 500 ml Kationenaustauscher Lewatit SP 112 WS (H-Form) gefüllt war. Nach dem Aufbringen der Produkte wurde mit 2 1 entionisiertem Wasser nachgespült. Anschließend wurde die Säule mit 1,51 6,5 %iger Ammoniaklösung eluiert. Das ablaufende Eluat wurde aufgefangen und anschließend am Rotationsverdampfer bei 55°C und 15 mbar eingeengt. Es wurden ca. 12 g aufgereinigte Fraktionen 1 bis 3 erhalten.

### Beispiel 2

### Herstellung von Silylverbindungen

0,4 Mol Hydroxyverbindung wurden in 400 ml getrocknetem tert.-Butylmethylether gelöst. Zu dieser Lösung wurden innerhalb von 30 min 32,6 g Trimethylchlorsilan und 48,42 g Hexamethylendisilazan zugetropft. Das Gemisch wurde solange gerührt (17 h) bis keine OH-Bande im IR-Spektrum sichtbar war. Der weiße Niederschlag wurde abfiltriert und die Lösung 3 mal mit je 400 ml Wasser extrahiert. Die organische Phase wurde abgetrennt, mit Magnesiumsulfat getrocknet und bei 40°C im Wasserstrahlvakuum vom Lösungsmittel befreit, wobei die Silylverbindung in der unten angegebenen Menge als farbloses Öl erhalten wurde.

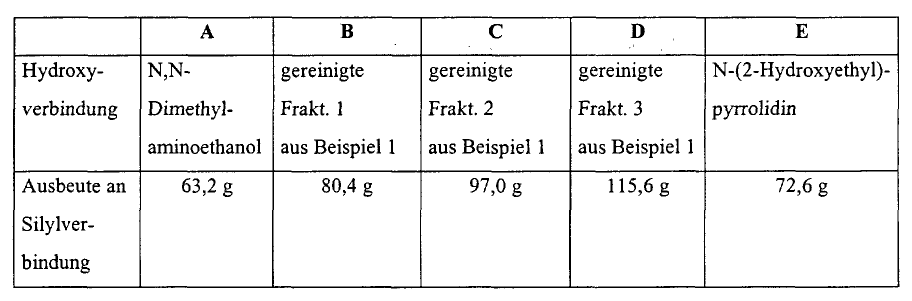

### Beispiel 3

### Herstellung erfindungsgemäßer Isocyanate (Verbindungen 1 bis 4 aus Tabelle 1)

Jeweils 0,2 Mol der Silylverbindungen aus Beispiel 2 wurden in einem Rührgefäß unter Feuchtigkeitsausschluss vorgelegt. Bei 25°C wurden 35,12 g Isocyanatocapronsäurechlorid innerhalb von 30 min. zugetropft. Dabei stieg die Temperatur auf 45 bis 55°C. Es wurde unter reduziertem Druck von 250 mbar angelegt und das Gemisch 7 h bei 90°C und dann 16 h bei 110°C gerührt. Das gebildete Trimethylchlorsilan wurde in einer Kühlfalle aufgefangen und ausgewogen. Im Reaktionsgefäß blieben die Verbindung 1 bis 4 aus Tabelle 1 in den unten angegebenen Mengen als hochviskose Öle zurück. Die Charakterisierung der Verbindungen erfolgte durch Elementaranalyse und NMR-Spektroskopie.

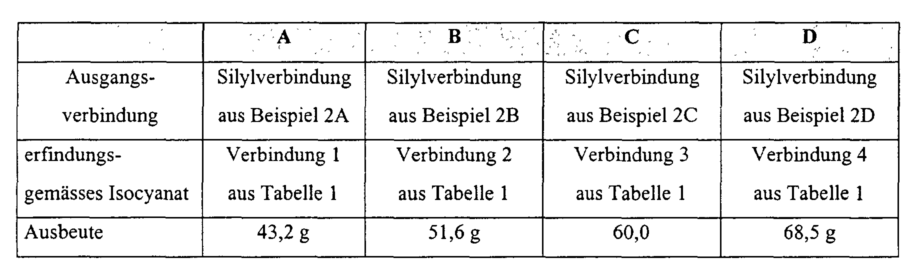

### Beispiel 4

### Herstellung eines erfindungsgemäßen Isocyanats

0,2 Mol der Silylverbindungen aus Beispiel 2D wurden wie in Beispiel 3 beschrieben mit 35,12 g Isocyanatocapronsäurechlorid umgesetzt. Man erhielt 48,0 g hochviskoses Öl, welches durch Elementaranalyse und NMR-Spektroskopie als Verbindung 7 der Tabelle 1 identifiziert wurde.

### Beispiel 5

### Herstellung eines Perlpolymerisates mit OH-Gruppen

In einem 250 ml-Glasreaktor wurden 120ml entionisiertes Wasser vorgelegt und hierin 1,5 g Dinatriumhydrogenphosphatdekahydrat, und 5 g Polyvinylalkohol (Verseifungsgrad 88 %) bei Raumtemperatur gelöst. Unter Rühren mit 450 rpm wurde eine Mischung aus 10,5 g Methylmethacrylat, 4 g Hydroxyethylmethacrylat, 0,5 g Ethylenglycoldimethacrylat 0,3 g Azobuttersäuredinitril und 37,5 g Chloroform zugesetzt. In das Reaktionsgefäß wurde ein Stickstoffstrom von 6 l/h eingeleitet. Der Ansatz wurde 21 h bei 70°C gehalten und dann auf Raumtemperatur abgekühlt. Danach wurde das Reaktionsgemisch in Bechergläser überführt und mit 1 l Wasser verdünnt. Das gebildete Perlpolymerisat setzte sich innerhalb von 4 h vollständig ab. Die überstehende Flüssigkeit wurde abdekantiert und verworfen. Das Perlpolymerisat wurde 4 mal mit je 1 l Wasser und dann 2 mal mit je Aceton gewaschen und bei Raumtemperatur im Vakuum bis zur Gewichtskonstanz getrocknet. Man erhielt 13,5 g Perlpolymerisat mit einer mittleren Teilchengröße von 28 um und einer OH- Zahl 115 mg KOH/g.

### Beispiel 6

### Modififizierung eines Perlpolymerisates

10 g Perlpolymerisat aus Beispiel 4 und 10 mg Dibutylzinndilaurat wurden in 100 ml getrocknetem Chloroform aufgeschlämmt und 10 h zum Quellen stehen gelassen. Zu dieser Suspension wurden 4,18 g Isocyanat aus Beispiel 3B auf einmal zugegeben und die Mischung solange bei 40°C gerührt bis kein Isocyanat IR-spektroskopisch nachweisbar war (22 h). Nach dem Abkühlen wurde das Perlpolymerisat durch Filtration isoliert, 2 mal mit je 200 ml Chlorofom und 2 mal mit je 200 ml Aceton gewaschen und bei Raumtemperatur im Vakuum bis zur Gewichtskonstanz getrocknet. Man erhielt 13,1 g Perlpolymerisat. Die Titration mit 0,1n Salzsäure ergab 1,1 mMol stark basische Gruppen/g.

## Patentansprüche

1. Isocyanate der Formel (I) 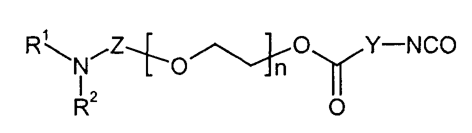 worin
R¹ und R² unabhängig von einander Alkyl mit 1 bis 6 C-Atomen,
wobei R¹ und R² zu einem Ring verbunden sein können,
Z Alkylen mit 2 bis 6 C-Atomen
n eine ganze Zahl von 0 bis 20
Y Alkylen mit 2 bis 6 C-Atomen bedeuten.

2. Isocyanate der Formel (II) 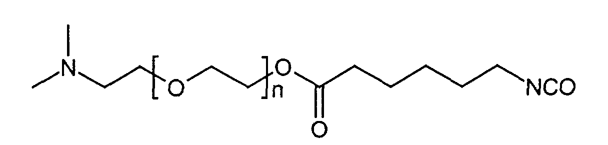 worin
n eine ganze Zahl von 0 bis 20 bedeutet.

3. Verfahren zur Herstellung von Isocyanaten nach Anspruch 1, **dadurch gekennzeichnet, dass** man Silylverbindungen der Formel (III) 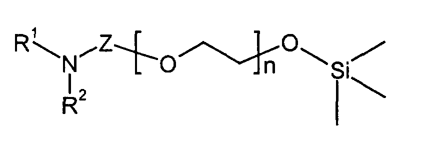 mit Isocyanatocarbonsäurechloriden der Formel (IV) umsetzt, 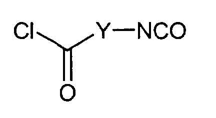 wobei R¹, R², Z, n, und Y die oben angegebenen Bedeutungen haben.

4. Umsetzungsprodukte von Isocyanaten nach Anspruch 1 oder 2 mit OH-, NH-, oder NH₂-funktionellen Polymeren.

5. Umsetzungsprodukte von Isocyanaten nach Anspruch 1 oder 2 mit OH-, NH-, oder NH₂-funktionellen Perlpolymerisaten

6. Verwendung der Umsetzungsprodukte aus Anspruch 5 zur Isolierung von Nukleinsäuren.
